# EUROPEAN PATENT SPECIFICATION

(11) **EP 3 366 765 B1**
(45) Date of publication and mention of the grant of the patent: **15.12.2021**
(21) Application number: 16857099.2
(22) Date of filing: 19.10.2016
(51) Int. Cl.: C12M 1/34, G01N 33/483

(54) **ELECTROCHEMICAL MEASUREMENT SYSTEM**
SYSTEM ZUR ELEKTROCHEMISCHEN MESSUNG
SYSTÈME DE MESURE ÉLECTROCHIMIQUE

(30) Priority: 22.10.2015 JP 2015207703
(43) Date of publication of application: 29.08.2018
(73) Proprietor: Panasonic Intellectual Property Management Co., Ltd., Osaka-shi, Osaka 540-6207 (JP)
(72) Inventor: USHIO, Hiroshi, Osaka-shi, Osaka 540-6207 (JP); YASUMI, Masahiro, Osaka-shi, Osaka 540-6207 (JP)
(74) Representative: Grünecker Patent- und Rechtsanwälte PartG mbB
(86) International application number: PCT/JP2016/004619
(87) International publication number: WO 2017/068778

(56) References cited:
- WO-A1-2014/073195
- WO-A1-2016/047114
- JP-A- 2003 532 116
- JP-A- 2004 108 863
- JP-A- 2004 108 971
- JP-A- 2005 532 060
- JP-A- 2009 002 839
- JP-A- 2009 195 107
- JP-A- 2010 121 948
- JP-A- 2012 063 287
- JP-A- 2013 011 482
- JP-A- 2015 099 070
- US-A1- 2002 025 584
- US-B2- 7 981 362
- DATE, Y. ET AL.: 'Monitoring oxygen consumption of single mouse embryos using an integrated electrochemical microdevice' BIOSENS. BIOELECTRON. vol. 30, 2011, pages 100 - 106, XP028334470
- SHIKU, H. ET AL.: 'Respiration activity of single bovine embryos entrapped in a cone- shaped microwell monitored by scanning electrochemical microscopy' ANAL. CHIM. ACTA vol. 522, 2004, pages 51 - 58, XP004549833
- OBREGON, R. ET AL.: 'A Pt layer/Pt disk electrode configuration to evaluate respiration and alkaline phosphatase activities of mouse embryoid bodies' TALANTA vol. 94, 2012, pages 30 - 35, XP028489254
- KOSUKE INO ET AL.: 'Denki Kagakuteki Shuho o Mochiita Saibo Kassei Hyoka' JOURNAL OF THE SOCIETY FOR BIOSCIENCE AND BIOENGINEERING, JAPAN vol. 92, 2014, pages 176 - 179, XP009505726
- JUN'ICHIRO ARAI ET AL.: 'Sanso Denkyokuho ni yoru Biseibutsu no Kokyu Sokutei o Shihyo to shita Yakuzai Screening' ELECTROCHEMISTRY vol. 67, no. 5, 1999, pages 479 - 483, XP009505724

## Description

### TECHNICAL FIELD

The present disclosure relates to an electrochemical measurement device and an electrochemical measurement system for performing electrochemical measurement of a biological sample, such as a cell.

### BACKGROUND ART

As a method for examining the activity state of a biological sample such as a fertilized ovum, a respiratory activity measurement method using electrochemical measurement is known.

For example, a respiratory activity measuring apparatus for measuring the respiratory activity of an embryo includes a chip for respiration measurement and an analysis unit. The chip for respiration measurement includes a substrate in which an electrode is disposed, a plurality of wells for introducing an embryo, and a micro passage. One embryo is introduced into each of the wells. A counter electrode and a reference electrode are interposed inside the well into which the embryo is introduced. In electrochemical measurement, a measurement potential is applied to a working electrode, the counter electrode, and the reference electrode of the chip for respiration measurement. The analysis unit calculates the amount of oxygen consumed by the embryo from current values measured before and after the introduction of the embryo in a state in which the potential is applied to the chip for respiration measurement. The respiratory activity measuring apparatus determines the respiratory activity and activity state of the embryo from the calculated amount of oxygen consumed by the embryo.

PTL 1 discloses a respiratory activity measuring apparatus similar to the above-mentioned respiratory activity measuring apparatus.

PTL2 discloses a plate with multiple wells for containing samples of biological liquids, each well comprising electrodes at its bottom.

PTL3 discloses a sensor with two wells that are connected to each other via channels and a sample holder, each well comprising electrodes at its bottom.

### CITATION LIST

### PATENT LITERATURE

PTL 1: Japanese Patent Laid-Open Publication No. 2010-121948
PTL2: US7981362
PTL3: US2002/025584

### SUMMARY

An electrochemical measurement system according to the invention is defined in claim 1.

This electrochemical measurement system can perform electrochemical measurement in plural wells in a short time. Further advantageous embodiments are defined in the dependent claims.

### BRIEF DESCRIPTION OF DRAWINGS

FIG. 1 is a perspective view of an electrochemical measurement device according to an exemplary embodiment.
FIG. 2 is a top view of the electrochemical measurement device according to the embodiment.
FIG. 3 is a cross-sectional view of the electrochemical measurement device along line III-III shown in FIG. 2.
FIG. 4 is a cross-sectional view of the electrochemical measurement device along line IV-IV shown in FIG. 2.
FIG. 5 is an enlarged cross-sectional view of the electrochemical measurement device illustrated in FIG. 4.
FIG. 6 is a top view of wells of the electrochemical measurement device according to the embodiment.
FIG. 7 is a schematic diagram of an electrochemical measurement system according to the embodiment.
FIG. 8 is a top view of another electrochemical measurement device according to the embodiment.
FIG. 9 is a top view of still another electrochemical measurement device according to the embodiment.
FIG. 10 is a perspective view of a further electrochemical measurement device according to the embodiment.
FIG. 11 is a top view of a further electrochemical measurement device according to the embodiment.
FIG. 12 is a top view of a further electrochemical measurement device according to the embodiment.
FIG. 13 is an enlarged cross-sectional view of a further electrochemical measurement device according to the embodiment.
FIG. 14 is a cross-sectional view of a further electrochemical measurement device according to the embodiment.
FIG. 15 is a cross-sectional view of a further electrochemical measurement device according to the embodiment.

### DETAIL DESCRIPTION OF EXEMPLARY EMBODIMENT

Hereinafter, an electrochemical measurement system according to embodiments of the present invention will be described in detail with reference to the drawings. It should be noted that the embodiments described below represent specific preferred examples of the electrochemical measurement device used in the system of the present invention. Therefore, numerical values, shapes, materials, constituents, and the arrangement and connection of the constituents, each being mentioned in the following embodiments, are merely exemplary, and are not intended to limit the scope of the present invention. Thus, among the constituents in the following embodiments, constituents not recited in the independent claim, which indicates the broadest concept of the present invention, are described as arbitrary constituents.

The drawings are schematic diagrams, and are not necessarily strictly made. In the drawings, constituents having substantially the same configuration are assigned the same reference signs, and duplicate descriptions of the constituents are omitted or simplified.

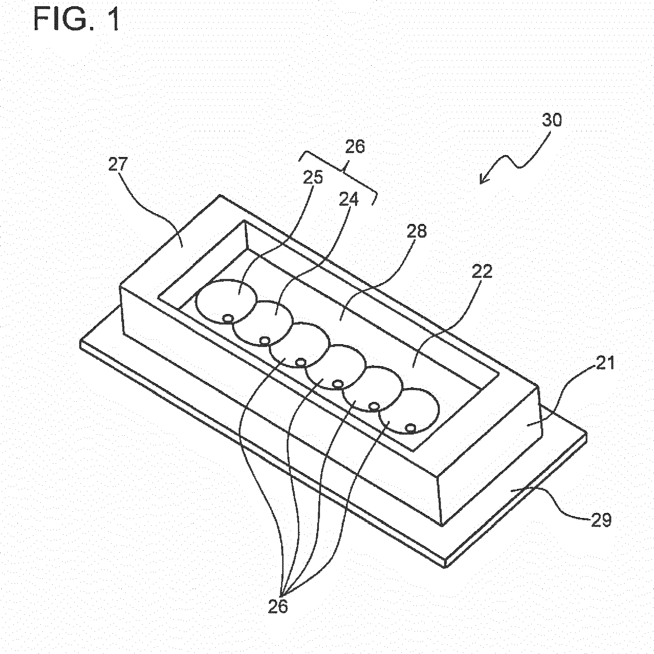

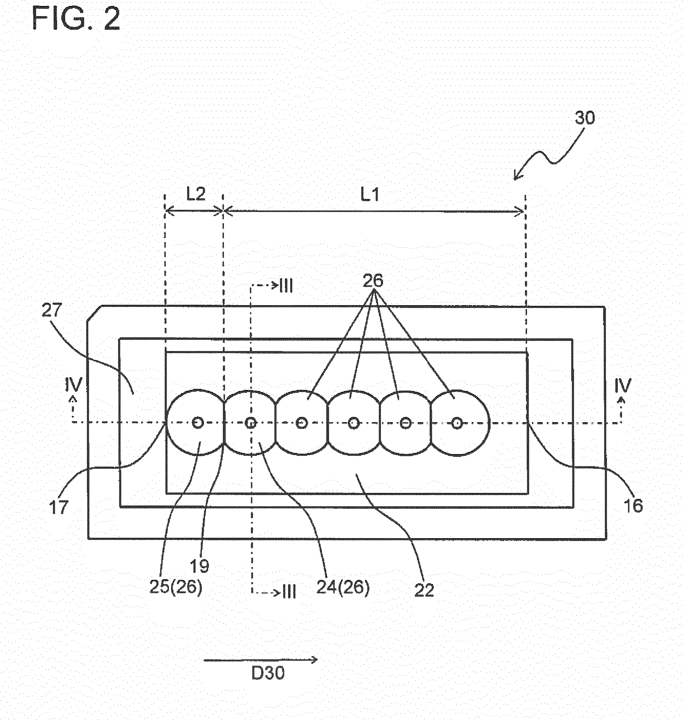

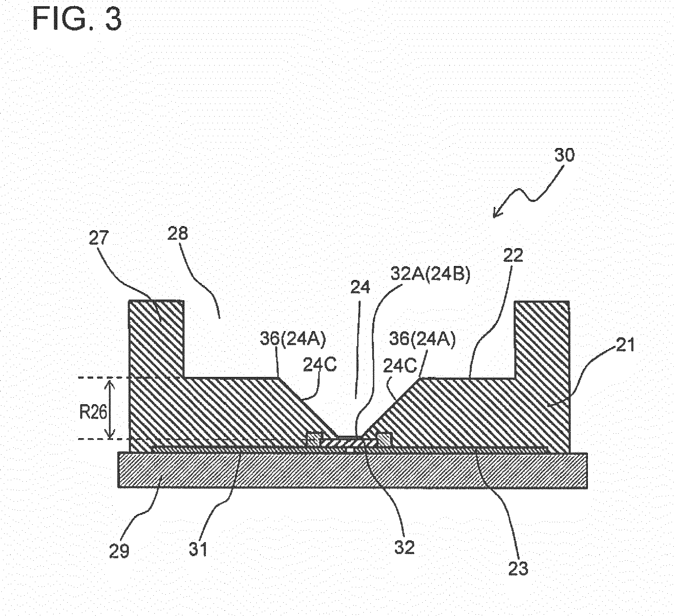

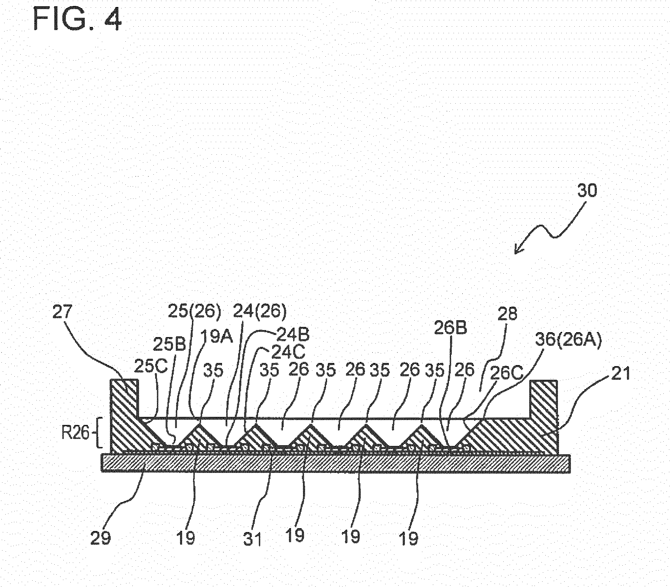

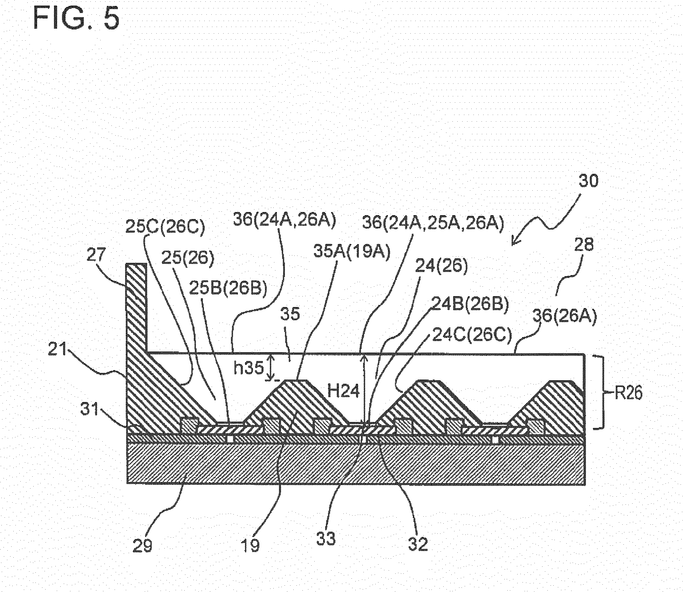

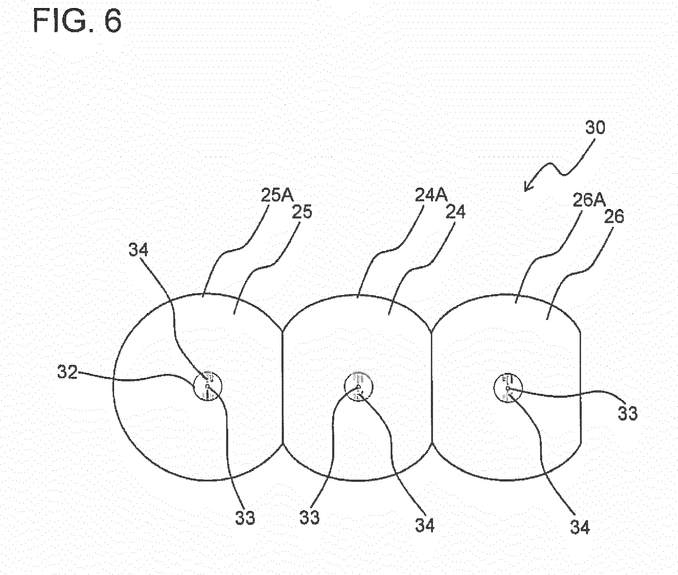

FIG. 1 and FIG. 2 are a perspective view and a top view of electrochemical measurement device 30 according to an embodiment, respectively. FIG. 3 is a cross-sectional view of electrochemical measurement device 30 along line III-III shown in FIG. 2. FIG. 4 is a cross-sectional view of electrochemical measurement device 30 along line IV-IV shown in FIG. 2. FIG. 5 is an enlarged cross-sectional view of electrochemical measurement device 30 illustrated in FIG. 4. FIG. 6 is an enlarged top view of electrochemical measurement device 30.

Electrochemical measurement device 30 is used for electrochemical measurement of objects, such biological samples. The biological sample may be cells and tissues, such as an embryo, that are collected from a living body. Electrochemical measurement device 30 is used for measuring, e.g. a respiratory activity of an embryo.

Electrochemical measurement device 30 includes plate 21 having upper surface 22, well 24 provided in upper surface 22 of plate 21, well 25 formed in upper surface 22 of plate 21 at a position different from a position of well 24, wall 19 separating dividing well 24 from well 25, placement portion 33 provided on bottom surface 24B of well 24, and measuring electrode 34 provided on bottom surface 24B of well 24. Plate 21 has communicating passage 35 provided therein between top end 36 at the outer edge of well 24 and bottom surface 24B of well 24. Communicating passage 35 spatially connects well 24 to well 25. Inside well 25, a counter electrode is configured to be disposed.

Plate 21 further has lower surface 23 opposite to upper surface 22. Plural wells 26 including wells 24 and 25 are provided in upper surface 22 of plate 21. In FIG. 1, six wells 26 are provided. Plate 21 is made of, for example, glass, resin, silicon, or ceramics.

Plate 21 includes wall 19 provided between wells 24 and 25. Wall 19 separates well 24 from well 25. Specifically, wall 19 separates bottom surface 24B of well 24 from bottom surface 25B of well 25. Wall 19 prevents a biological sample introduced into well 24 from moving to well 25. Furthermore, wall 19 divides a measuring liquid contained in wells 24 and 25 into a portion of the measuring liquid contained in well 24 and a portion of the measuring liquid contained in well 25. Thus, wall 19 can reduce impacts of metabolites and other substances derived from a biological sample placed in well 24 on measurement conducted in well 25.

Frame 27 is provided at the outer peripheral portion of upper surface 22 of plate 21. Frame 27 is formed by, for example, integral molding with plate 21 or cutting processing. Reservoir 28 surrounded by frame 27 is provided above the wells 26. Electrochemical measurement device 30 may not necessarily include frame 27 or reservoir 28.

Bottom plate 29 is provided below lower surface 23 of plate 21. Bottom plate 29 is made of, for example, glass, resin, silicon, or ceramics. Circuit board 31 and electrode chip 32 are provided above bottom plate 29. Plate 21 and bottom plate 29 may be unitarily formed by integral molding.

Electrode chip 32 is provided below well 26. Upper surface 32A of electrode chip 32 constitutes bottom surface 26B of well 26. Placement portion 33 and measuring electrodes 34 are disposed on upper surface 32A of electrode chip 32. Placement portion 33 is configured to have a biological sample placed thereon.

Placement portion 33 is implemented by, for example, a recess provided in upper surface 32A of electrode chip 32. The shape of placement portion 33 is appropriately determined according to a biological sample to be measured. Placement portion 33 may be implemented by a flat portion of upper surface 32A of electrode chip 32, for example.

Measuring electrodes 34 are provided around placement portion 33. Measuring electrodes 34 are located away from placement portion 33 by different distances. Measuring electrodes 34 are made of, for example, metal, such as platinum, gold, or silver. Alternatively, measuring electrodes 34 may be made of conductive material, such as carbon or lithium cobalt oxide. The material of measuring electrodes 34 may be selected in consideration of, for example, the composition of the measuring liquid, a voltage necessary for measurement, or an impact on the biological sample.

Wells 26 are recesses formed in plate 21 and electrode chip 32.

Wells 26 including well 24 has, for example, inner wall surface 26C inclining downward with respect to upper surface 22 of plate 21 toward the center of well 26 from the outer edge thereof. Inner wall surface 26C is connected to outer edge 26A and bottom surface 26B of well 26. Well 24 has outer edge 24A located at upper surface 22 of plate 21, bottom surface 24B, and inner wall surface 24C connected to outer edge 24A and bottom surface 24B. Well 25 has outer edge 25A located at upper surface 22 of plate 21, bottom surface 25B, and inner wall surface 25C connected to outer edge 25A and bottom surface 25B.

A through-hole is provided in the bottom of well 26. Placement portion 33 and measuring electrodes 34 of electrode chip 32 are exposed from the through-hole. In other words, the upper surface of electrode chip 32 constitutes bottom surface 26B of well 26. Measuring electrodes 34 contact a measuring liquid contained in well 26. The biological sample to be placed in well 26 is introduced onto placement portion 33 from above electrochemical measurement device 30.

Circuit board 31 includes a wiring. Measuring electrodes 34 are electrically connected to the wiring of circuit board 31. Circuit board 31 allows the wiring of electrochemical measurement device 30 to be easily designed.

Electrochemical measurement device 30 includes a connection unit to be connected to an external device, such as an electrochemical measuring apparatus. The connection unit is provided, for example, around electrochemical measurement device 30 or on a lower surface of electrochemical measurement device 30.

Electrochemical measurement device 30 includes communicating passage 35 spatially connecting wells 26 to each other. Communicating passage 35 is located in region R26 in a height direction in which wells 26 are provided.

Communicating passage 35 will be particularly described below.

Well 24 is spatially connected to well 25 via communicating passage 35. Communicating passage 35 is provided in region R26 in the height direction between top end 36 at outer edge 24A and bottom surface 24B of well 24 into which the sample is introduced. Wells 24 and 25 are spatially connected to each other, hence allowing the measuring liquid to flow between wells 24 and 25 via communicating passage 35.

In electrochemical measurement device 30, upper surface 19A of wall 19 is located below upper surface 22 of plate 21. In other words, communicating passage 35 is located above wall 19, and upper surface 19Aof wall 19 faces communicating passage 35. Thus, electrochemical measurement device 30 can ensure an electrical connection between wells 24 and 25 in a region below top end 36 at outer edge 24A of well 24 via the measuring liquid having conductivity. Top end 36 at outer edge 24A of well 24 is a boundary between well 24 and upper surface 22 of plate 21.

Distance h35 between lower surface 35A of communicating passage 35 and upper surface 22 of plate 21 is equal to or smaller than one third of distance H24 between upper surface 22 of plate 21 and bottom surface 24B of well 24.

Communicating passage 35 is preferably positioned so as to prevent the biological sample from moving between wells 24 and 25.

An operation of electrochemical measurement device 30 will be described below.

In accordance with the embodiment, an embryo is employed as the biological sample.

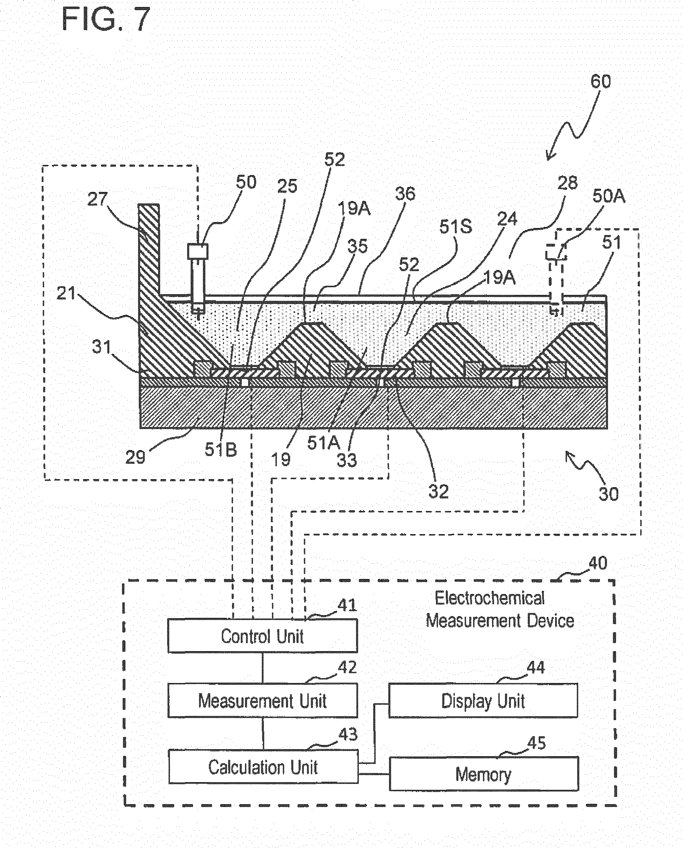

FIG. 7 is a block diagram of electrochemical measurement system 60, for schematically illustrating the operation of electrochemical measurement device 30.

The electrochemical measuring system 60 of the present invention includes electrochemical measurement device 30 and electrochemical measuring apparatus 40. Electrochemical measurement device 30 is connected to electrochemical measuring apparatus 40 via the connection unit.

Measuring liquid 51 is poured into electrochemical measurement device 30 from above. Measuring liquid 51 is poured so that liquid surface 51S of measuring liquid 51 is located above upper surface 19A of wall 19. Upper surface 19A is located below top end 36 at outer edge 24A of well 24 of electrochemical measurement device 30. Thus, portion 51A of conductive measuring liquid 51 contained in well 24 is electrically connected to portion 51B of measuring liquid 51 contained in well 25.

Next, biological samples 52, e.g. embryos, are introduced onto respective placement portions 33 of wells 26.

One biological sample 52 is introduced into one well 26. Bottom surfaces 26B (24B, 25B) of wells 26 (24, 25) have respective placement portions 33 each configured to have biological sample 52 placed thereon.

Subsequently, counter electrode 50 is inserted into well 25 so as to contact the measuring liquid. Counter electrode 50 is made of, for example, noble metal, such as platinum, gold, or silver. The material of counter electrode 50 is selected in consideration of the composition of measuring liquid 51 in the measurement, and a voltage and a current required for the measurement.

For the purpose of determining the potentials of measuring electrodes 34 more accurately, reference electrode 50A may be provided so as to contact measuring liquid 51. Reference electrode 50A is made of, for example, noble metal, such as platinum, gold, or silver. The material of reference electrode 50A is selected in consideration of the composition of measuring liquid 51 in the measurement and a voltage and a current required for the measurement. Electrochemical measurement system 60 may not necessarily include reference electrode 50A. In this case, counter electrode 50 may function as reference electrode 50A.

Electrochemical measuring apparatus 40 includes control unit 41, measuring unit 42, and calculation unit 43.

Control unit 41 is configured to apply a measurement potential to measuring electrode 34 and counter electrode 50.

For example, for an electrochemical measurement in well 24, a measurement potential is applied between well 24 and counter electrode 50. The applied potential causes an oxidation-reduction current to flow between measuring electrodes 34 of well 24 and counter electrode 50 disposed in well 25.

Measuring unit 42 is configured to measure the oxidation-reduction current flowing between measuring electrodes 34 of well 24 and counter electrode 50.

Calculation unit 43 is configured to calculate a respiratory activity value of biological sample 52 based on the measured oxidation-reduction current.

Similarly, electrochemical measurement in well 25 can be performed by measuring an oxidation-reduction current flowing between
measuring electrodes 34 of well 25 and counter electrode 50.

Control unit 41, measuring unit 42, and calculation unit 43 are implemented by, for example, circuits including a sensor and a semiconductor. Control unit 41, measuring unit 42, and calculation unit 43 may be independently configured or may be integrally configured.

Electrochemical measuring apparatus 40 may include, for example, display unit 44 for displaying information, such as measured current values and calculation results, and memory unit 45 for storing such information.

As described above, electrochemical measurement device 30 performs electrochemical measurement in plural wells 26 with using one counter electrode 50 with a small amount of measuring liquid 51. Therefore, it is not necessary to fill electrochemical measurement device 30 with measuring liquid 51 to top end 36 at outer edge 24A of well 24.

In the above-mentioned conventional chip for respiration measurement, when electrochemical measurement is performed in each of plural wells, a counter electrode and a reference electrode necessary for the measurement are inserted into the wells. This inserting of the counter electrode and the reference electrode into the wells into which a biological sample is introduced needs to be carefully performed in consideration of positions of the electrodes with respect to the biological sample. Thus, when the biological samples introduced into the wells are sequentially measured, an operator needs to perform the insertion and extraction of the counter electrode and the working electrode into and from the wells. The operator needs to repeat such troublesome operation. Hence, the use of this chip for respiration measurement increases a time to perform electrochemical measurement.

In the electrochemical measurement device 30 of the system 40 according to the present invention, when biological samples 52 introduced into respective wells 26 are measured, it is not necessary to move counter electrode 50 at every measurement in wells 26, accordingly reducing operation burdens on an operator. Thus, electrochemical measurement device 30 can perform electrochemical measurement in a shorter time.

In the measurement, counter electrode 50 is an obstacle to operation. Therefore, well 25 in which counter electrode 50 is disposed is preferably located outside well 24. This arrangement reduces operation burdens on the operator.

Well 24 is disposed from well 25 in predetermined direction D30, as illustrated in FIG. 2. Distance L1 between wall 19 and end 16 of plate 21 in predetermined direction D30 is larger than distance L2 between wall 19 and end 17 of plate 21 opposite to end 16 in predetermined direction D30.

In the case where frame 27 is provided on upper surface 22 of plate 21, the end portion of plate 21 is the boundary between upper surface 22 and the inner surface of frame 27.

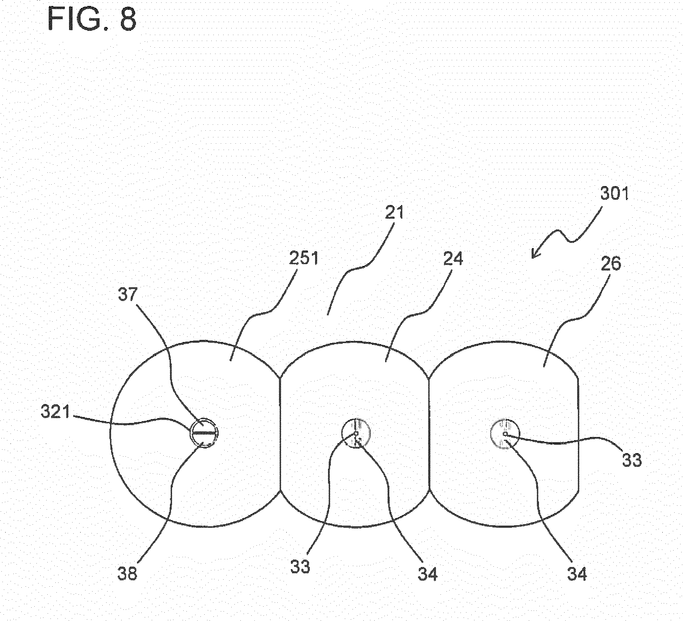

FIG. 8 is a top view of another electrochemical measurement device 301 according to the embodiment. In FIG. 8, components identical to those of electrochemical measurement device 30 illustrated in FIGs. 1 to 7 are denoted by the same reference numerals. In electrochemical measurement device 301 illustrated in FIG. 8, plate 21 has well 251 therein instead of well 25. Electrochemical measurement device 301 includes counter electrode 37 and reference electrode 38 which are provided on the bottom surface of well 251. Counter electrode 37 has a semicircular shape. Reference electrode 38 has a semicircular shape.

Counter electrode 37 and reference electrode 38 are provided on the upper surface of electrode chip 321 located below well 251. Counter electrode 37 and reference electrode 38 are exposed from a through-hole formed in the bottom of a recess of plate 21. In other words, counter electrode 37 and reference electrode 38 contact measuring liquid 51.

Well 251 has neither a measuring electrode nor a placement portion therein. In other words, electrochemical measurement device 301 does not perform electrochemical measurement of a biological sample in well 251.

In electrochemical measurement in well 24, electrochemical measurement device 301 measures an oxidation-reduction current flowing between counter electrode 37 and measuring electrode 34 of well 24.

As described above, counter electrode 37 provided in well 251 allows electrochemical measurement device 301 to perform electrochemical measurement in plural wells 26 other than well 251. Electrochemical measurement device 301 does not require counter electrode 50 inserted in the measurement.

This configuration reduces burdens on the operator, and decreases a time required for the measurement. Counter electrode 50 is generally expensive. Hence, counter electrode 50 is repeatedly used in electrochemical measurement. Such repetitive usage of counter electrode 50 may contaminate counter electrode 50. Contaminated counter electrode 50 may be an obstacle against stable measurement. Electrochemical measurement device 30 according to the embodiment employs disposable counter electrode 37, and therefore, can perform the measurement more stably.

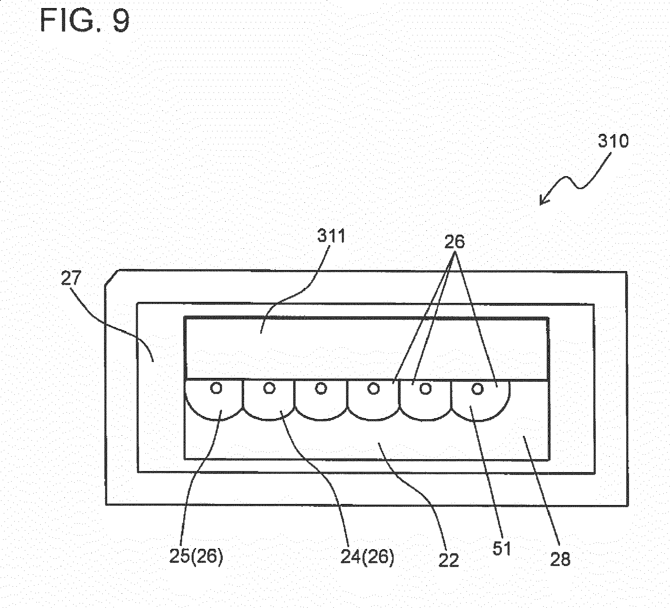

FIG. 9 is a top view of still another electrochemical measurement device 310 according to the embodiment. In FIG. 9, components identical to those of electrochemical measurement device 30 illustrated in FIGs. 1 to 7 are denoted by the same reference numerals.

Electrochemical measurement device 310 further includes cover 311 partially covering wells 24 and 25. Cover 311 prevents measuring liquid 51 contained in well 26 from leaking to reservoir 28. During the measurement, biological sample 52 is introduced into and taken out of electrochemical measurement device 310. Therefore, in consideration of operability for an operator, cover 311 preferably partially covers wells 26. In other words, wells 26 are preferably partially exposed from cover 311. In accordance with the embodiment, cover 311 covers about 40% to 50% of each well 26.

Cover 311 is fixed on upper surface 22 of plate 21. Cover 311 is arranged such that placement portion 33 provided inside each of wells 26 is exposed viewing from above. Cover 311 does not overlap placement portion 33, and allows the operator to introduce biological sample 52 while observing biological sample 52 from above with a microscope.

In the case where cover 311 is made of transparent material, cover 311 may cover the entirety of wells 26. In this case, biological sample 52 is introduced and taken out by removing cover 311.

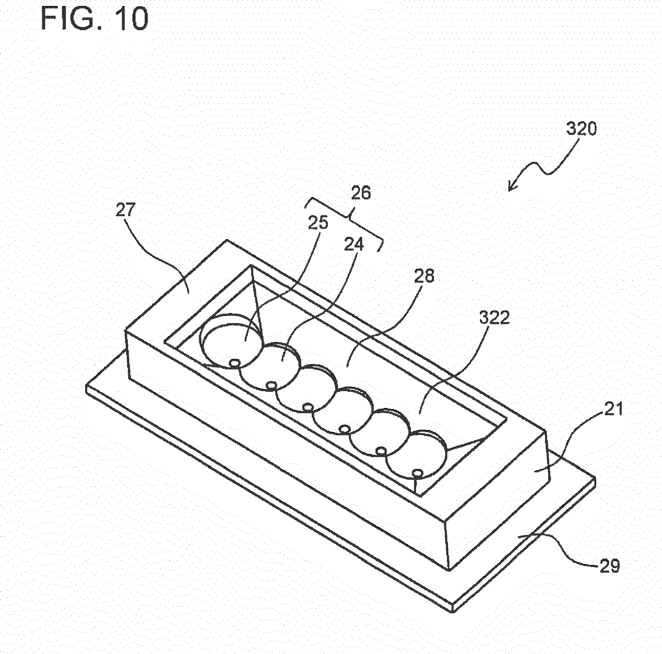

FIG. 10 is a perspective view of further electrochemical measurement device 320 according to the embodiment. In FIG. 10, components identical to those of electrochemical measurement device 30 illustrated in FIGs. 1 to 7 are denoted by the same reference numerals.

Upper surface 322 of electrochemical measurement device 320 inclines downward toward well 24 and well 25 from the outer periphery of plate 21.

Inclining upper surface 322 allows measuring liquid 51 jumped out of well 26 to flow back into wells 26. This configuration prevents measuring liquid 51 from decreasing due to the jumping-out of measuring liquid 51.

### (Modification 1)

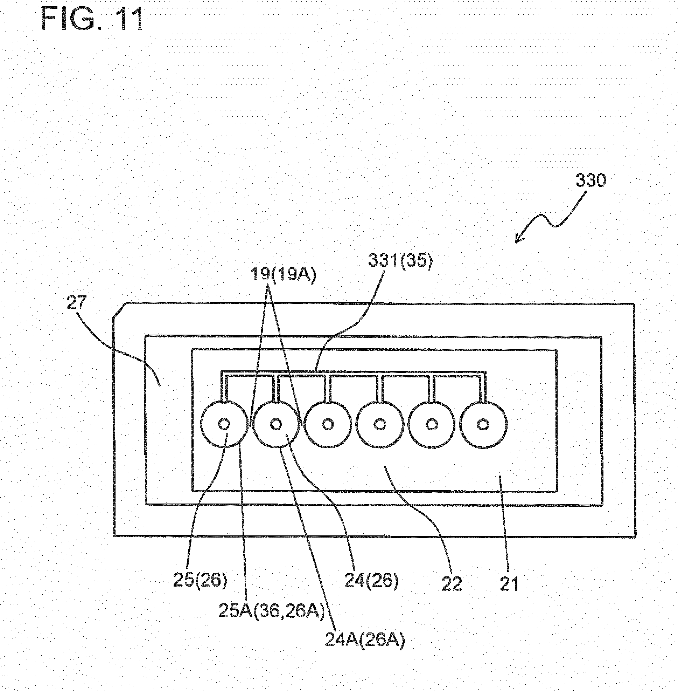

FIG. 11 is a top view of electrochemical measurement device 330 according to Modification 1. In FIG. 11, components identical to those of electrochemical measurement device 30 illustrated in FIGs. 1 to 7 are denoted by the same reference numerals.

In electrochemical measurement device 330, groove 331 is formed in upper surface 22 of plate 21.

Groove 331 is formed in upper surface 22 of plate 21. In other words, the bottom surface of groove 331 is located below top end 36 at outer edge 24A of well 24.

Groove 331 is connected to plural wells 26 including wells 24 and 25.

Groove 331 functions as communicating passage 35 for spatially connecting well 24 to well 25.

Groove 331 does not overlap wall 19 provided between plural wells 26. In other words, upper surface 19A of wall 19 between wells 24 and 25 is located at the same height as top end 36 at outer edge 24A of well 24. This configuration prevents measuring liquid 51 and biological sample 52 from moving between wells 24 and 25. Upper surface 19A of wall 19 provided between wells 24 and 25 may be located above the top end of outer edge 24A of well 24. This configuration prevents measuring liquid 51 and biological sample 52 from moving between wells 24 and 25.

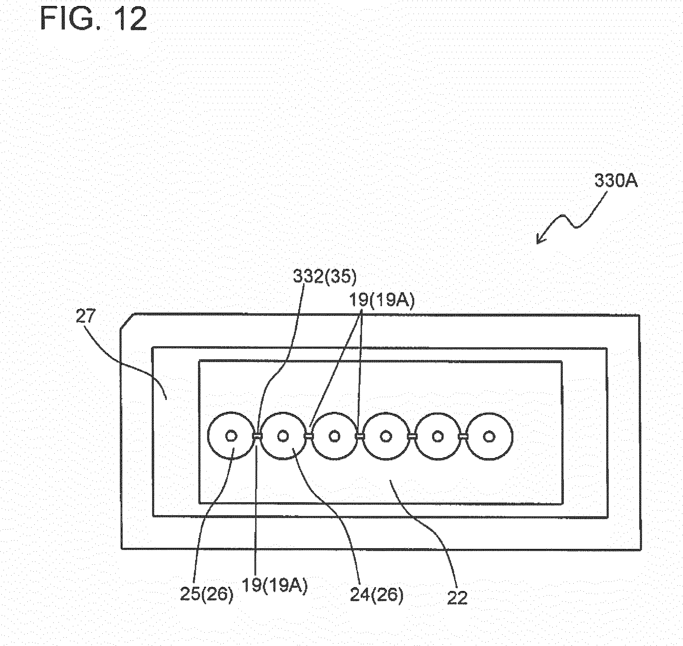

FIG. 12 is a top view of another electrochemical measurement device 330A according to Modification 1. In FIG. 12, components identical to those of electrochemical measurement device 330 illustrated in FIG. 11 are denoted by the same reference numerals. In electrochemical measurement device 330A illustrated in FIG. 12, groove 332 serving as communicating passage 35 is formed in upper surface 19A of wall 19 separating well 24 from well 25.

This configuration allows electrochemical measurement device 330A to perform electrochemical measurement in plural wells 26 with single counter electrode 50 with a small amount of measuring liquid 51.

### (Modification 2)

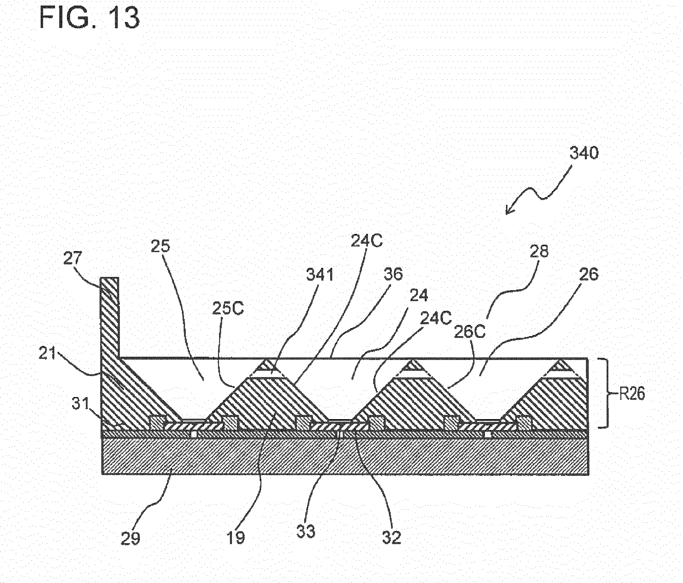

FIG. 13 is a cross-sectional view of electrochemical measurement device 340 according to Modification 2. In FIG. 13, components identical to those of electrochemical measurement device 30 illustrated in FIGs. 1 to 7 are denoted by the same reference numerals.

In electrochemical measurement device 340, through-hole 341 is formed in wall 19 separating well 24 from well 25. Through-hole 341 penetrates from inner wall surface 24C of well 24 to inner wall surface 25C of well 25. In other words, through-hole 341 penetrates from inner wall surface 26C of one well 26 to inner wall surface 26C of another well 26.

Through-hole 341 is formed in wall 19. Through-hole 341 is provided in region R26 between top end 36 at outer edge 24A of well 24 and bottom surface 24B of well 24 in the height direction. In other words, the upper surface of through-hole 341 is located below top end 36 of outer edge 24A of well 24. Furthermore, the lower surface of through-hole 341 is located above bottom surface 24B of well 24.

Through-hole 341 serves as communicating passage 35 spatially connecting well 24 to well 25.

This configuration allows electrochemical measurement device 340 to perform electrochemical measurement in plural wells 26 with single counter electrode 50 with a small amount of measuring liquid 51.

The electrochemical measurement device may not necessarily include circuit board 31 or electrode chip 32. For example, a recess formed in the plate and having no through-hole may serve as well 26.

In this case, placement portion 33 and measuring electrodes 34 are formed on the bottom surface of the recess. Furthermore, placement portion 33 and measuring electrodes 34 may be provided on bottom plate 29.

Measuring liquid 51 may fill up the inside of reservoir 28 surrounded by frame 27 of electrochemical measurement device 30. This configuration allows counter electrode 50 to be electrically connected via measuring liquid 51 to measuring electrodes 34 (and working electrodes) disposed in each of wells 26. This configuration allows electrochemical measurement device 30 to measure biological samples 52 introduced into respective wells 26 with using single counter electrode 50. In this case, counter electrode 50 contacts measuring liquid 51, and is inserted into, for example, the inside of well 26 or reservoir 28.

Measuring liquid 51 in electrochemical measurement device 30 flows when electrochemical measurement device 30 moves or vibrates. When measuring liquid 51 fills up reservoir 28 above the level of top end 36 of outer edge 26A of well 26, the amount of measuring liquid 51 is increased, and accordingly, a large amount of measuring liquid 51 flows between plural wells 26. Such flow of measuring liquid 51 may causes biological sample 52 to float from placement portion 33 and move. Biological sample 52 often has a small size, for example, ranging from 50 µm to 300 µm. Therefore, the movement of biological sample 52 caused by the flow of measuring liquid 51 may cause a problem, such as out of sight of biological sample 52.

To avoid such a problem, measuring liquid 51 preferably fills the electrochemical measurement device so as not to exceed the level of top end 36 of outer edge 26A of well 26. As described above, in electrochemical measurement device 30, well 24 is connected with well 25 in region R26 between top end 36 at outer edge 24A of well 24 and bottom surface 24B of well 24. Therefore, even a small amount of measuring liquid 51 allows a current to flow between measuring electrodes 34 and each of counter electrodes 50 and 37 disposed in well 25. By performing measurement using a small amount of measuring liquid 51, electrochemical measurement device 30 can prevent the movement of biological sample 52 due to the flow of measuring liquid 51. An operator can perform electrochemical measurement of the biological sample with a small amount of measuring liquid 51, and therefore, can perform measurement without losing sight of biological sample 52.

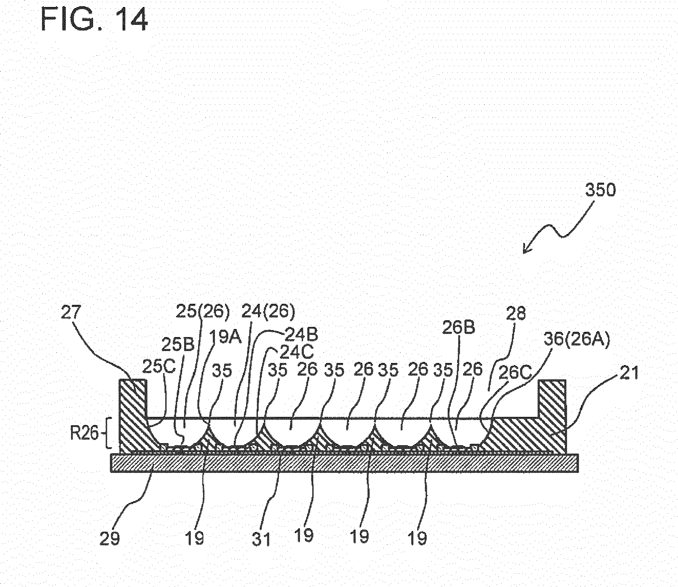

FIG. 14 is a cross-sectional view of further electrochemical measurement device 350 according to the embodiment. In FIG. 14, components identical to those of electrochemical measurement device 30 illustrated in FIGs. 1 to 7 are denoted by the same reference numerals. In electrochemical measurement device 350 illustrated in FIG. 14, inner wall surfaces 26C (24C, 25C) of wells 26 (24, 25) are concave toward outside wells 26 (24, 25).

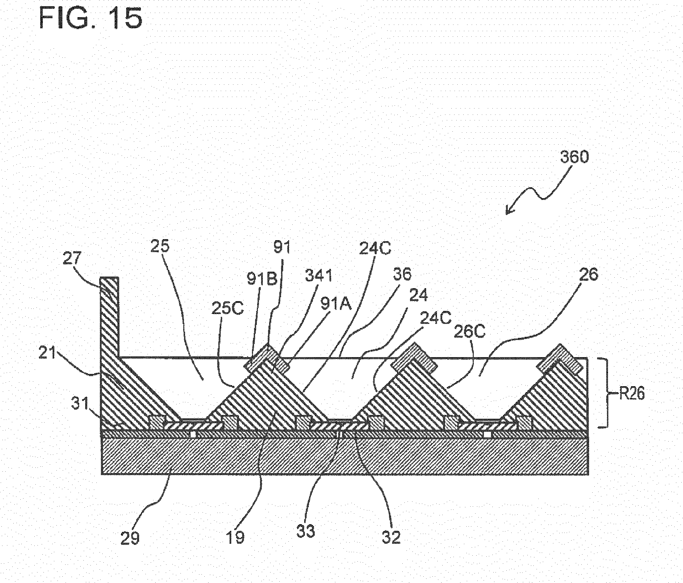

FIG. 15 is a cross-sectional view of further electrochemical measurement device 360 according to the embodiment. In FIG. 15, components identical to those of electrochemical measurement device 30 illustrated in FIGs. 1 to 7 are denoted by the same reference numerals. Electrochemical measurement device 360 further includes electrode 91 connecting well 24 to well 25 to ensure electrical connection between wells 24 and 25. Electrode 91 includes end portions 91A and 91B. End portion 91A of electrode 91 is located on inner wall surface 24C between outer edge 24A of well 24 and bottom surface 24B of well 24. End portion 91B of electrode 91 is located on inner wall surface 25C between outer edge 25A of well 25 and bottom surface 25B of well 25. Electrode 91 is away from bottom surfaces 24B and 25B (26B) of wells 24 and 25 (26), but may reach bottom surfaces 24B and 25B (26B) of wells 24 and 25 (26). Electrode 91 is made of, for example, the same material as measuring electrodes 34.

In electrochemical measurement device 360, a portion of the measuring liquid contained in well 24 (26) may not necessarily contact a portion of the measuring liquid contained in well 25 (26), and may be separated from the portion of the measuring liquid contained in well 25 (26). Portion 91A of electrode 91 contacts the portion of the measuring liquid contained in well 24 while portion 91B of electrode 91 is electrically connected to the portion of the measuring liquid contained in well 25. This configuration allows the portion of the measuring liquid contained in well 24 to be electrically connected via electrode 91 to the portion of the measuring liquid contained in well 25.

Communicating passage 35 of electrochemical measurement device 30, groove 331 of electrochemical measurement device 330, through-hole 341 of electrochemical measurement device 340, and electrode 91 of electrochemical measurement device 360 constitute conductive passages electrically connecting between portion 51A of measuring liquid 51 contained in well 24 and portion 51B of measuring liquid 51 contained in well 25.

Up to this point, the electrochemical measurement system has been described on the basis of the embodiment and the modifications, but the present invention is not limited thereto. Various modifications to the embodiment may be conceivable by those skilled in the art without departing from the scope of the invention as defined by the appended claims.

In the embodiment, terms, such as "upper surface", "lower surface", "above", and "below", indicating directions indicate relative directions determined only by the relative positional relationship of constituent components of the electrochemical measurement device, and do not indicate absolute directions, such as a vertical direction.

### INDUSTRIAL APPLICABILITY

An electrochemical measurement system according to the present disclosure are particularly useful as a device for examining and analyzing activity of biological samples.

### REFERENCE MARKS IN THE DRAWINGS

19 wall
21 plate
22, 322 upper surface
23 lower surface
24 well (first well)
24A outer edge
24B bottom surface
24C inner wall surface
25, 251 well (second well)
25A outer edge
25B bottom surface
25C inner wall surface
26 well
26A outer edge
26B bottom surface
26C inner wall surface
27 frame
28 reservoir
29 bottom plate
30, 301, 310, 320, 330, 340 electrochemical measurement device
31 circuit board
32, 321 electrode chip
37 counter electrode
38 reference electrode
40 electrochemical measuring apparatus
41 control unit
42 measuring unit
43 calculation unit
44 display unit
45 memory unit
50 counter electrode
60 electrochemical measurement system
311 cover
331, 332 groove
341 through-hole

## Claims

1. An electrochemical measurement system for electrochemically measuring a biological sample with using a measuring liquid having conductivity, the electrochemical measurement system comprising:
an electrochemical measurement device (30, 301, 310, 320, 330, 330A, 340, 350, 360); and
an electrochemical measuring apparatus (40),
wherein the electrochemical measurement device (30, 301, 310, 320, 330, 330A, 340, 350, 360) includes:
a plate (21) having an upper surface (22, 322), the plate (21) having a first well (24) and a second well (25, 251) which are provided in the upper surface (22, 322) at positions different from each other, the first well (24) having a bottom surface (24B), the second well (25, 251) having a bottom surface (25B), the plate (21) including a wall (19) separating the bottom surface (24B) of the first well (24) from the bottom surface (25B) of the second well (25, 251);
a measuring electrode (34) disposed inside the first well (24);
a counter electrode (37) disposed inside the second well (25, 251); and
a placement portion (33) provided on the bottom surface (24B) of the first well (24), the placement portion (33) being configured to have the biological sample placed thereon,
wherein the measuring electrode (34) is provided around the placement portion (33) on the bottom surface (24B) of the first well (24),
wherein the first well (24) and the second well (25, 251) are configured to contain the measuring liquid therein,
wherein, when the first well (24) and the second well (25, 251) contain the measuring liquid therein, a portion of the measuring liquid contained in the first well (24) is electrically connected to a portion of the measuring liquid contained in the second well (25, 251), wherein the electrochemical measuring apparatus (40) includes:
a control unit (41) that is configured to apply a potential to the measuring electrode (34);
a measuring unit (42) that is configured to measure a current flowing in the measuring electrode (34); and
a calculation unit (43) that is configured to calculate an amount of an activity of the biological sample based on the measured current,
wherein the plate (21) has a communicating passage (35, 331, 341) therein, the communicating passage (35, 331, 341) communicating with the first well (24) above the bottom surface (24B) of the first well (24) to allow the first well (24) to communicate with the second well (25, 251), **characterized in that** a plurality of measuring electrodes (34) is provided around the placement portion (33).

2. The electrochemical measurement system according to claim 1,
wherein the first well (24) further has an outer edge (24A) and an inner wall surface (24C) connected to the outer edge (24A) and the bottom surface (24B) of the first well (24), and
wherein the communicating passage (35, 331, 341) opens to the inner wall surface (24C) of the first well (24) above the bottom surface (24B) of the first well (24) to allow the first well (24) to communicate with the second well (25, 251), and when the communicating passage (35, 331, 341) is filled with the measuring liquid, the communicating passage (35, 331, 341) electrically connects the portion of the measuring liquid contained in the first well (24) to the portion of the measuring liquid contained in the second well (25, 251) upon being filled with the measuring liquid.

3. The electrochemical measurement system according to claim 2, wherein the communicating passage (35, 331, 341) is located above the wall (19).

4. The electrochemical measurement system according to claim 2, wherein the communicating passage comprises a through-hole (341) provided in the wall (19).

5. The electrochemical measurement system according to claim 2, wherein the communicating passage comprises a groove (331) provided in the upper surface (22, 322) of the plate (21) so as to connect the first well (24) to the second well (25, 251).

6. The electrochemical measurement system according to claim 5, wherein the groove (331) is provided in an upper surface (19A) of the wall (19).

7. The electrochemical measurement system according to claim 1, wherein the counter electrode (37) is provided on the bottom surface (25B) of the second well (25, 251).

8. The electrochemical measurement system according to claim 1, further comprising a working electrode provided inside the second well (25, 251),
wherein the bottom surface (25B) of the second well (25, 251) includes a second placement portion configured to have the biological sample placed thereon, and wherein the working electrode is provided on the bottom surface (25B) of the second well (25, 251) around the second placement portion.

9. The electrochemical measurement system according to claim 1, wherein the upper surface (22, 322) of the plate (21) inclines downward toward the first well (24) and the second well (25, 251) from outside the upper surface (22, 322) of the plate (21).

10. The electrochemical measurement system according to claim 1, further comprising a frame (27) provided at an outer periphery of the upper surface (22, 322) of the plate (21).

11. The electrochemical measurement system according to claim 1, further comprising a cover (311) provided on the upper surface (22, 322) of the plate (21) so as to cover the first well (24) and the second well (25, 251).

12. The electrochemical measurement system according to claim 11, wherein the first placement portion of the first well (24) is exposed from the cover (311) viewing from above.

13. The electrochemical measurement system according to claim 1,
wherein the first well (24) further has an inner wall surface (24C) extending from an outer edge (24A) of the first well (24) to the bottom surface (24B) of the first well (24), and wherein the inner wall surface (24C) of the first well (24) is concave toward outside the first well (24).

14. The electrochemical measurement system according to claim 1,
wherein the first well (24) further has an inner wall surface (24C) extending from an outer edge (24A) of the first well (24) to the bottom surface (24B) of the first well (24), and
wherein the inner wall surface (24C) of the first well (24) inclines with respect to the upper surface (22, 322) of the plate (21).

15. The electrochemical measurement system according to claim 1,
wherein the second well (25, 251) is disposed in a predetermined direction from the first well (24), and
wherein a distance between the wall (19) and an end of the plate (21) in the predetermined direction is larger than a distance between the wall (19) and another end of the plate (21) opposite to the end of the plate (21) in the predetermined direction.

16. The electrochemical measurement system according to claim 1, wherein a distance between a bottom surface of the communicating passage (35) and the upper surface (22, 322) of the plate (21) is equal to or smaller than one third a distance between the bottom surface (24B) of the first well (24) and the upper surface (22, 322) of the plate (21).

17. The electrochemical measurement system according to claim 1, further comprising
an electrode including a first portion located inside the first well (24) and a second portion located inside the second well (25, 251),
wherein the first portion of the electrode contacts the portion of the measuring liquid contained in the first well (24), and the second portion of the electrode contacts the portion of the measuring liquid contained in the second well (25, 251), thereby electrically connecting the portion of the measuring liquid contained in the first well (24) with the portion of the measuring liquid contained in the second well (25, 251).

## Patentansprüche

1. Elektrochemisches Messungssystem zum elektrochemischen Messen einer biologischen Probe unter Einsatz einer Messflüssigkeit, die Leitfähigkeit aufweist, wobei das elektrochemische Messungssystem umfasst:
eine elektrochemische Messungsvorrichtung (30, 301, 310, 320, 330, 330A, 340, 350, 360); sowie
eine elektrochemische Messeinrichtung (40),
wobei die elektrochemische Messungsvorrichtung (30, 301, 310, 320, 330, 330A, 340, 350, 360) enthält:
eine Platte (21) mit einer oberen Fläche (22, 322), wobei die Platte (21) eine erste Mulde (24) und eine zweite Mulde (25, 251) aufweist, die in der oberen Fläche (22, 322) an voneinander verschiedenen Positionen vorhanden sind, die erste Mulde (24) eine Bodenfläche (24B) hat, die zweite Mulde (25, 251) eine Bodenfläche (25B) hat und die Platte (21) eine Wand (19) enthält, die die Bodenfläche (24B) der ersten Mulde (24) von der Bodenfläche (25B) der zweiten Mulde (25, 251) trennt;
eine Messelektrode (34), die im Inneren der ersten Mulde (24) angeordnet ist;
eine Gegenelektrode (37), die im Inneren der zweiten Mulde (25, 251) angeordnet ist; und
einen Auflegeabschnitt (33), der an der Bodenfläche (24B) der ersten Mulde (24) vorhanden ist, wobei der Auflegeabschnitt (33) so ausgeführt ist, dass die biologische Probe darauf aufgelegt wird,
wobei die Messelektrode (34) um den Auflegeabschnitt (33) herum an der Bodenfläche (24B) der ersten Mulde (24) vorhanden ist,
die erste Mulde (24) und die zweite Mulde (25, 251) so ausgeführt sind, dass die Messflüssigkeit darin aufgenommen wird,
wenn in der ersten Mulde (24) und der zweiten Mulde (25, 251) die Messflüssigkeit aufgenommen ist, ein Teil der in der ersten Mulde (24) aufgenommenen Messflüssigkeit elektrisch mit einem Teil der in der zweiten Mulde (25, 251) aufgenommenen Messflüssigkeit verbunden ist,
und die elektrochemische Messeinrichtung (40) einschließt:
eine Steuer-Einheit (41), die so ausgeführt ist, dass sie ein Potenzial an die Messelektrode (34) anlegt;
eine Mess-Einheit (42), die so ausgeführt ist, dass sie einen in der Messelektrode (34) fließenden Strom misst; sowie
eine Berechnungs-Einheit (43), die so ausgeführt ist, dass sie ein Maß einer Aktivität der biologischen Probe auf Basis des gemessenen Stroms berechnet,
wobei die Platte (21) einen Verbindungskanal (35, 331, 341) darin aufweist, und der Verbindungskanal (35, 331, 341) mit der ersten Mulde (24) oberhalb der Bodenfläche (24B) der ersten Mulde (24) in Verbindung steht, um zuzulassen, dass die erste Mulde (24) mit der zweiten Mulde (25, 251) in Verbindung steht, **dadurch gekennzeichnet, dass**
eine Vielzahl von Messelektroden (34) um den Auflegeabschnitt (33) herum vorhanden ist.

2. Elektrochemisches Messungssystem nach Anspruch 1,
wobei die erste Mulde (24) des Weiteren einen äußeren Rand (24A) und eine innere Wandfläche (24C) aufweist, die mit dem äußeren Rand (24A) und der Bodenfläche (24B) der ersten Mulde (24) verbunden sind, und
der Verbindungskanal (35, 331, 341) sich zu der inneren Wandfläche (24C) der ersten Mulde (24) oberhalb der Bodenfläche (24B) der ersten Mulde (24) öffnet, um zuzulassen, dass die erste Mulde (24) mit der zweiten Mulde (25, 251) in Verbindung steht, und, wenn der Verbindungskanal (35, 331, 341) mit der Messflüssigkeit gefüllt ist, der Verbindungskanal (35, 331, 341) den in der ersten Mulde (24) aufgenommenen Teil der Messflüssigkeit elektrisch mit dem in der zweiten Mulde (25, 251) aufgenommenen Teil der Messflüssigkeit verbindet, sobald er mit der Messflüssigkeit gefüllt ist.

3. Elektrochemisches Messungssystem nach Anspruch 2, wobei sich der Verbindungskanal (35, 331, 341) oberhalb der Wand (19) befindet.

4. Elektrochemisches Messungssystem nach Anspruch 2, wobei der Verbindungskanal ein in der Wand (19) vorhandenes Durchgangsloch (341) umfasst.

5. Elektrochemisches Messungssystem nach Anspruch 2, wobei der Verbindungskanal eine Nut (331) umfasst, die in der oberen Fläche (22, 322) der Platte (21) vorhanden ist, um die erste Mulde (24) mit der zweiten Mulde (25, 251) zu verbinden.

6. Elektrochemisches Messungssystem nach Anspruch 5, wobei die Nut (331) in einer oberen Fläche (19A) der Wand (19) vorhanden ist.

7. Elektrochemisches Messungssystem nach Anspruch 1, wobei die Gegenelektrode (37) an der Bodenfläche (25B) der zweiten Mulde (25, 251) vorhanden ist.

8. Elektrochemisches Messungssystem nach Anspruch 1, das des Weiteren umfasst:
eine Arbeitselektrode, die im Inneren der zweiten Mulde (25, 251) vorhanden ist,
wobei die Bodenfläche (25B) der zweiten Mulde (25, 251) einen zweiten Auflegeabschnitt enthält, der so ausgeführt ist, dass die biologische Probe darauf aufgelegt wird, und
die Arbeitselektrode an der Bodenfläche (25B) der zweiten Mulde (25, 251) um den zweiten Auflegeabschnitt herum vorhanden ist.

9. Elektrochemisches Messungssystem nach Anspruch 1, wobei die obere Fläche (22, 322) der Platte (21) von außerhalb der oberen Fläche (22, 322) der Platte (21) nach unten zu der ersten Mulde (24) und der zweiten Mulde (25, 251) hin geneigt ist.

10. Elektrochemisches Messungssystem nach Anspruch 1, das des Weiteren einen Rahmen (27) umfasst, der an einem Außenumfang der oberen Fläche (22, 322) der Platte (21) vorhanden ist.

11. Elektrochemisches Messungssystem nach Anspruch 1, das des Weiteren eine Abdeckung (311) umfasst, die an der oberen Fläche (22, 322) der Platte (21) vorhanden ist und die erste Mulde (24) und die zweite Mulde (25, 251) abdeckt.

12. Elektrochemisches Messungssystem nach Anspruch 11, wobei der erste Auflegeabschnitt der ersten Mulde (24), von oben gesehen, von der Abdeckung (311) frei ist.

13. Elektrochemisches Messungssystem nach Anspruch 1,
wobei die erste Mulde (24) des Weiteren eine innere Wandfläche (24C) aufweist, die sich von einem äußeren Rand (24A) der ersten Mulde (24) zu der Bodenfläche (24B) der ersten Mulde (24) erstreckt, und
die innere Wandfläche (24C) der ersten Mulde (24) zur Außenseite der ersten Mulde (24) hin konkav ist.

14. Elektrochemisches Messungssystem nach Anspruch 1,
wobei die erste Mulde (24) des Weiteren eine innere Wandfläche (24C) aufweist, die sich von einem äußeren Rand (24A) der ersten Mulde (24) zu der Bodenfläche (24B) der ersten Mulde (24) erstreckt, und
die innere Wandfläche (24C) der ersten Mulde (24) in Bezug auf die obere Fläche (22, 322) der Platte (21) geneigt ist.

15. Elektrochemisches Messungssystem nach Anspruch 1,
wobei die zweite Mulde (25, 251) in einer vorgegebenen Richtung von der ersten Mulde (24) aus angeordnet ist, und
ein Abstand zwischen der Wand (19) und einem Ende der Platte (21) in der vorgegebenen Richtung größer ist als ein Abstand zwischen der Wand (19) und einem anderen Ende der Platte (21) gegenüber dem Ende der Platte (21) in der vorgegebenen Richtung.

16. Elektrochemisches Messungssystem nach Anspruch 1, wobei ein Abstand zwischen einer Bodenfläche des Verbindungskanals (35) und der oberen Fläche (22, 322) der Platte (21) genauso groß ist wie oder kleiner als ein Drittel eines Abstandes zwischen der Bodenfläche (24B) der ersten Mulde (24) und der oberen Fläche (22, 322) der Platte (21).

17. Elektrochemisches Messungssystem nach Anspruch 1, das des Weiteren umfasst:
eine Elektrode, die einen ersten Abschnitt, der sich im Inneren der ersten Mulde (24) befindet, und einen zweiten Abschnitt enthält, der sich im Inneren der zweiten Mulde (25, 251) befindet,
wobei der erste Abschnitt der Elektrode mit dem in der ersten Mulde (24) aufgenommenen Teil der Messflüssigkeit in Kontakt kommt, und der zweite Abschnitt der Elektrode mit dem in der zweiten Mulde (25, 251) aufgenommenen Teil der Messflüssigkeit in Kontakt kommt, wodurch der in der ersten Mulde (24) aufgenommene Teil der Messflüssigkeit elektrisch mit dem in der zweiten Mulde (25, 251) aufgenommenen Teil der Messflüssigkeit verbunden wird.

## Revendications

1. Système de mesure électrochimique pour le mesurage électrochimique d'un échantillon biologique au moyen d'un liquide de mesure ayant une conductivité, le système de mesure électrochimique comprenant :
un dispositif de mesure électrochimique (30, 301, 310, 320, 330, 330A, 340, 350, 360) ; et
un appareil de mesure électrochimique (40),
le dispositif de mesure électrochimique (30, 301, 310, 320, 330, 330A, 340,350, 360) comprenant :
une plaque (21) ayant une surface supérieure (22, 322), la plaque (21) ayant un premier puits (24) et un deuxième puits (25, 251) qui sont ménagés dans la surface supérieure (22, 322) à des positions différentes l'une de l'autre, le premier puits (24) ayant une surface inférieure (24B), le deuxième puits (25, 251) ayant une surface inférieure (25B), la plaque (21) incluant une paroi (19) séparant la surface inférieure (24B) du premier puits (24) de la surface inférieure (25B) du deuxième puits (25, 251) ;
une électrode de mesure (34) disposée à l'intérieur du premier puits (24) ;
une contre-électrode (37) disposée à l'intérieur du deuxième puits (25, 251) ; et
une partie de placement (33) ménagée sur la surface inférieure (24B) du premier puits (24), la partie de placement (33) étant configurée pour que l'échantillon biologique soit placé sur celle-ci,
l'électrode de mesure (34) étant située autour de la partie de placement (33) sur la surface inférieure (24B) du premier puits (24),
le premier puits (24) et le deuxième puits (25, 251) étant configurés pour contenir le liquide de mesure dans leur intérieur,
où, quand le premier puits (24) et le deuxième puits (25, 251) contiennent le liquide de mesure dans leur intérieur, une partie du liquide de mesure contenu dans le premier puits (24) est connectée électriquement à une partie du liquide de mesure contenue dans le deuxième puits (25, 251),
l'appareil de mesure électrochimique (40) incluant :
un module de commande (41) qui est configuré pour appliquer un potentiel à l'électrode de mesure (34) ;
un module de mesure (42) qui est configuré pour mesurer un courant circulant dans l'électrode de mesure (34) ; et
un module de calcul (43) qui est configuré pour calculer un degré d'une activité de l'échantillon biologique sur la base du courant mesuré,
la plaque (21) ayant un passage de communication (35, 331, 341) dans on intérieur, le passage de communication (35, 331, 341) communiquant avec le premier puits (24) au-dessus de la surface inférieure (24B) du premier puits (24) pour permettre au premier puits (24) de communiquer avec le deuxième puits (25, 251),
**caractérisé en ce qu'**une pluralité d'électrodes de mesure (34) est située autour de la partie de placement (33).

2. Système de mesure électrochimique selon la revendication 1,
dans lequel le premier puits (24) comporte en outre un bord extérieur (24A) et une surface de paroi intérieure (24C) reliée au bord extérieur (24A) et à la surface inférieure (24B) du premier puits (24), et
dans lequel le passage de communication (35, 331, 341) s'ouvre sur la surface de paroi intérieure (24C) du premier puits (24) au-dessus de la surface inférieure (24B) du premier puits (24) pour permettre au premier puits (24) de communiquer avec le deuxième puits (25, 251), et quand le passage de communication (35, 331, 341) est rempli du liquide de mesure, le passage de communication (35, 331, 341) connecte électriquement la partie du liquide de mesure contenue dans le premier puits (24) à la partie du liquide de mesure contenue dans le deuxième puits (25, 251) lors du remplissage avec le liquide de mesure.

3. Système de mesure électrochimique selon la revendication 2, dans lequel le passage de communication (35, 331, 341) est situé au-dessus de la paroi (19).

4. Système de mesure électrochimique selon la revendication 2, dans lequel le passage de communication comprend un trou traversant (341) ménagé dans la paroi (19).

5. Système de mesure électrochimique selon la revendication 2, dans lequel le passage de communication comprend une rainure (331) ménagée dans la surface supérieure (22, 322) de la plaque (21) de façon à relier le premier puits (24) au deuxième puits (25, 251).

6. Système de mesure électrochimique selon la revendication 5, dans lequel la rainure (331) est ménagée dans une surface supérieure (19A) de la paroi (19).

7. Système de mesure électrochimique selon la revendication 1, dans lequel la contre-électrode (37) est située sur la surface inférieure (25B) du deuxième puits (25, 251).

8. Système de mesure électrochimique selon la revendication 1, comprenant en outre une électrode de travail située à l'intérieur du deuxième puits (25, 251),
la surface inférieure (25B) du deuxième puits (25, 251) incluant une seconde partie de placement configurée pour que l'échantillon biologique soit placé sur celle-ci, et
l'électrode de travail étant située sur la surface inférieure (25B) du deuxième puits (25, 251) autour de la seconde partie de placement.

9. Système de mesure électrochimique selon la revendication 1, dans lequel la surface supérieure (22, 322) de la plaque (21) s'incline vers le bas en direction du premier puits (24) et du deuxième puits (25, 251) depuis l'extérieur de la surface supérieure (22, 322) de la plaque (21).

10. Système de mesure électrochimique selon la revendication 1, comprenant en outre un cadre (27) situé au niveau d'une périphérie extérieure de la surface supérieure (22, 322) de la plaque (21).

11. Système de mesure électrochimique selon la revendication 1, comprenant en outre un couvercle (311) situé sur la surface supérieure (22, 322) de la plaque (21) de façon à couvrir le premier puits (24) et le deuxième puits (25, 251).

12. Système de mesure électrochimique selon la revendication 11, dans lequel la première partie de placement du premier puits (24) est exposée par rapport au couvercle (311), vu du dessus.

13. Système de mesure électrochimique selon la revendication 1,
dans lequel le premier puits (24) comporte en outre une surface de paroi intérieure (24C) s'étendant d'un bord extérieur (24A) du premier puits (24) à la surface inférieure (24B) du premier puits (24), et
dans lequel la surface de paroi intérieure (24C) du premier puits (24) est concave en direction de l'extérieur du premier puits (24).

14. Système de mesure électrochimique selon la revendication 1,
dans lequel le premier puits (24) comporte en outre une surface de paroi intérieure (24C) s'étendant d'un bord extérieur (24A) du premier puits (24) à la surface inférieure (24B) du premier puits (24), et
dans lequel la surface de paroi intérieure (24C) du premier puits (24) est inclinée par rapport à la surface supérieure (22, 322) de la plaque (21).

15. Système de mesure électrochimique selon la revendication 1,
dans lequel le deuxième puits (25, 251) est disposé dans une direction prédéfinie à partir du premier puits (24), et
dans lequel une distance entre la paroi (19) et une extrémité de la plaque (21) dans la direction prédéfinie est supérieure à une distance entre la paroi (19) et une autre extrémité de la plaque (21) opposée à l'extrémité de la plaque (21) dans la direction prédéfinie.

16. Système de mesure électrochimique selon la revendication 1, dans lequel une distance entre une surface inférieure du passage de communication (35) et la surface supérieure (22, 322) de la plaque (21) est inférieure ou égale à un tiers d'une distance entre la surface inférieure (24B) du premier puits (24) et la surface supérieure (22, 322) de la plaque (21).

17. Système de mesure électrochimique selon la revendication 1, comprenant en outre
une électrode incluant une première partie située à l'intérieur du premier puits (24) et une deuxième partie située à l'intérieur du deuxième puits (25, 251),
la première partie de l'électrode étant en contact avec la partie du liquide de mesure contenue dans le premier puits (24), et la deuxième partie de l'électrode étant en contact avec la partie du liquide de mesure contenue dans le deuxième puits (25, 251), ce qui permet de connecter électriquement la partie du liquide de mesure contenue dans le premier puits (24) à la partie du liquide de mesure contenue dans le deuxième puits (25, 251).
